# FASCICULE DE BREVET EUROPEEN

(11) **EP 1 194 084 B1**
(45) Date de publication et mention de la délivrance du brevet: **29.12.2004**
(21) Numéro de dépôt: 00949650.6
(22) Date de dépôt: 06.07.2000
(51) Int. Cl.: A61F 2/16

(54) **IMPLANT INTRAOCULAIRE**
INTRAOKULARES IMPLANTAT
INTRAOCULAR IMPLANT

(30) Priorité: 08.07.1999 FR 9908837
(43) Date de publication de la demande: 10.04.2002
(73) Titulaire: CORNEAL INDUSTRIE, 74370 Pringy (FR)
(72) Inventeur: BOS, Gilles, F-74330 La Balme de Sillingy (FR); GANTIN, Denis, F-74130 Bonneville (FR)
(74) Mandataire: Dronne, Guy
(86) Numéro de dépôt international: PCT/FR2000/001940
(87) Numéro de publication internationale: WO 2001/003610

(56) Documents cités:
- EP-A- 0 215 468
- EP-A- 0 391 452
- EP-A- 0 507 292
- WO-A-97/41805
- WO-A-98/56315
- FR-A- 2 668 922
- O. NISHI ET AL.: "Preventingposterior capsule opacification by creating a discontinuous sharp bend in the capsule" J.CATARACT REFRACTIVE SURG, vol. 25, avril 1999 (1999-04), pages 521-526, XP000900073 cité dans la demande

## Description

La présente invention concerne un implant intraoculaire du type "à bords carrés".

Les implants intraoculaires sont bien connus. Ils sont constitués essentiellement par une partie optique sensiblement circulaire et par une partie haptique qui sert au maintien de la partie optique à l'intérieur de l'oeil de telle manière que l'axe optique de la partie optique de l'implant coïncide avec l'axe optique de l'oeil. Les extrémités libres de la partie haptique sont en appui sur la paroi interne de l'oeil pour développer une force élastique de rappel assurant le maintien de l'implant.

Une des utilisations principales de tels implants intraoculaires consiste dans la mise en place de cet implant dans le sac capsulaire après l'ablation du cristallin lors d'une opération de la cataracte.

On sait que la prolifération cellulaire après la chirurgie de la cataracte est la principale complication post-opératoire de ce type de chirurgie. Cette prolifération cellulaire peut provoquer une opacification complète de la partie postérieure du sac capsulaire. Il est alors nécessaire de procéder à une capsulotomie en utilisant le laser ND-Yag.

Selon les données fournies par la littérature en cette matière, le taux de capsulotomies peut atteindre 50 % au bout de 3 ans après l'opération notamment avec des lentilles réalisées en matériau rigide du type PMMA.

Les études conduites notamment par Nishi et al. qui ont fait l'objet d'une publication dans la revue "Journal of Cataract Refract Surgery", volume 25 avril 1999 semblent indiquer qu'il est possible de bloquer la prolifération des cellules sur la capsule postérieure grâce à l'action du bord de la partie optique de l'implant sur la capsule postérieure lorsque cette partie optique comporte un bord "dit carré". Le terme de bord carré a été adopté pour définir les bords de partie optique dont la tranche forme avec la surface optique un angle voisin de 90° et qui a gardé un aspect vif.

Par ailleurs, on sait que dans les implants intraoculaires qui sont maintenant le plus souvent monoblocs, la partie haptique est reliée à la périphérie de la partie optique par une zone dite zone de raccordement. Par implant monobloc, il faut entendre un implant en une seule pièce, les parties optiques et haptiques pouvant être réalisées en des matériaux différents. Dans le cas des implants qui ont fait l'objet des expérimentations mentionnées ci-dessus la partie haptique est constituée par deux anses de largeur réduite de telle manière que les zones de raccordement ne constituent qu'un pourcentage très réduit de l'ensemble de la périphérie de la partie optique. On comprend que dans ce cas le bord carré de la partie optique assure un blocage efficace de la prolifération des cellules sur la capsule postérieure du fait même que ce bord optique carré n'est interrompu que dans des zones de longueurs très limitées correspondant aux zones de raccordement. Cependant, ces zones peuvent permettre la prolifération des cellules.

On comprend que le problème est encore beaucoup plus important dans le cas où ces zones de raccordement ou cette zone de raccordement représente un pourcentage significatif de la longueur totale de la périphérie de la partie optique. En effet, dans cette zone ou ces zones de raccordement, la prolifération ne pourra être bloquée en raison de l'absence de bords carrés de la partie optique. Des implants dont la ou les zones de raccordement représentent une partie non négligeable de la périphérie de la partie optique se rencontrent de plus en plus souvent notamment lorsque ces implants monoblocs sont réalisés en un matériau souple du type "hydrogel" ou du type "silicone". Ce type de zone de raccordement peut également se rencontrer avec des implants réalisés en matériau rigide du type PMMA par exemple lorsque la partie haptique de contact avec la paroi interne de l'oeil est constituée par une forme sensiblement en anneau raccordée à la partie optique par un seul bras sensiblement radial dont la largeur est nécessairement relativement importante pour assurer une liaison convenable entre la partie optique et l'anneau de contact de la partie haptique.

Il faut également rappeler que la pratique chirurgicale de mise en place de l'implant à l'intérieur de l'oeil tend à utiliser une incision dans la cornée de dimension de plus en plus réduite. Il est donc nécessaire, lors de la conception des implants intraoculaires aussi bien en ce qui concerne leur partie optique que leur partie haptique ou encore le raccordement de ces deux parties, de veiller à ce que l'épaisseur globale de l'implant reste réduite afin de permettre l'implantation de l'implant par une incision de dimension réduite. Cela est en particulier vrai mais non exclusivement dans le cas d'implants dont la partie optique est réalisée en un matériau souple permettant le pliage de cette partie optique autour d'un diamètre de celle-ci.

La demande de brevet européen EP 0 215. 468 décrit un cristallin artificiel en materiau souple comprenant une partie haptique et au moins deux anses haptiques. Chaque anse comporte dans sa zone de raccordement une surépaisseur postérieure.

Un objet de la présente invention est de fournir un implant intraoculaire pour sac capsulaire du type à bords carrés permettant donc de bloquer effectivement la prolifération cellulaire sur la capsule postérieure, en particulier dans le cas où la ou les zones de raccordement de la partie haptique à la partie optique présentent une longueur non négligeable tout en maintenant une épaisseur de l'implant aussi réduite que possible.

Pour atteindre ce but, selon l'invention, l'implant intraoculaire pour sac capsulaire comprend une partie optique présentant un dioptre antérieur et un dioptre postérieur, au moins un élément haptique, chaque élément haptique présentant une zone de raccordement à la périphérie de la partie optique :
- la partie optique comporte en outre, en dehors des zones de raccordement, une face latérale cylindrique de diamètre D1 raccordée au dioptre postérieur de la partie optique et parallèle à l'axe optique de l'implant, la longueur de la face latérale selon l'axe étant égale à h,
- le dioptre postérieur est limité par un cercle de diamètre D1,
- et il comprend en outre, dans chaque zone de raccordement, une extension radiale présentant une face antérieure, une face postérieure et une face latérale disposée sensiblement sur une surface réglée de diamètre D2 (D2 > D1) et présentant une longueur h' selon la direction de l'axe sensiblement égale à h,
- la face postérieure de chaque extension est disposée sur la calotte sphérique qui contient le dioptre postérieur,
- chaque élément haptique étant raccordé à la partie optique par la face antérieure de l'extension correspondante, à l'extérieur du dioptre antérieur, par quoi chaque extension constitue une marche réalisée par le décalage entre le dioptre postérieur de la partie optique et la zone de raccordement de l'élément haptique, la face latérale de chaque extension formant une portion de bord carré avec le dioptre postérieur.

On comprend que grâce à la présence des extensions radiales au niveau de la ou des zones de raccordement qui constituent par leurs parois latérales une marche résultant du décalage entre le dioptre postérieur de la partie optique et la zone de raccordement de l'élément haptique, on obtient la continuité du bord carré sur toute la périphérie de la partie optique. De plus, le fait que la "racine" de la ou des parties haptiques soit rattachée à la face antérieure de la ou des extensions évite une augmentation de l'épaisseur globale de l'implant.

Selon un mode préféré de mise en oeuvre, la calotte sphérique sur laquelle sont disposés le dioptre postérieur de la partie optique et les faces postérieures des extensions formant les marches a un rayon compris entre 11 et 13 mm.

En effet, les expérimentations effectuées pour la mise au point de la présente invention ont montré que c'était ce diamètre qui permettait le meilleur contact entre la capsule postérieure et le dioptre postérieur de l'implant empêchant ainsi la prolifération des cellules. On assure ainsi la tension de la capsule postérieure qui est très fine, de l'ordre de 5 microns, dans la zone limitée par le contact du bord carré de la lentille. On évite ainsi les risques de formation de plis dans la capsule postérieure et donc les risques de prolifération de cellules le long de ces plis.

De préférence encore, la ou les parties haptiques font avec le plan optique un angle a compris entre 5 et 12 degrés dirigé vers la face antérieure de l'implant.

Cette angulation tend à plaquer le dioptre postérieur de l'implant et la face postérieure des extensions formant marche contre la capsule postérieure.

D'autres caractéristiques et avantages de l'invention apparaîtront mieux à la lecture de la description qui suit de plusieurs modes de réalisation de l'invention donnés à titre d'exemples non limitatifs. La description se réfère aux figures annexées, sur lesquels :
- la figure 1a est une vue de face d'un premier implant intraoculaire conforme à l'invention ;
- la figure 1b est une vue de côté de l'implant de la figure 1a ;
- la figure 1c est une vue partielle de la figure 1b montrant plus en détail le raccordement entre la partie haptique et la partie optique de l'implant ;
- la figure 2a est une vue de face d'un deuxième mode de réalisation d'un implant à bords carrés en vue de face ; et
- la figure 2b est une vue de côté de l'implant de la figure 2a.

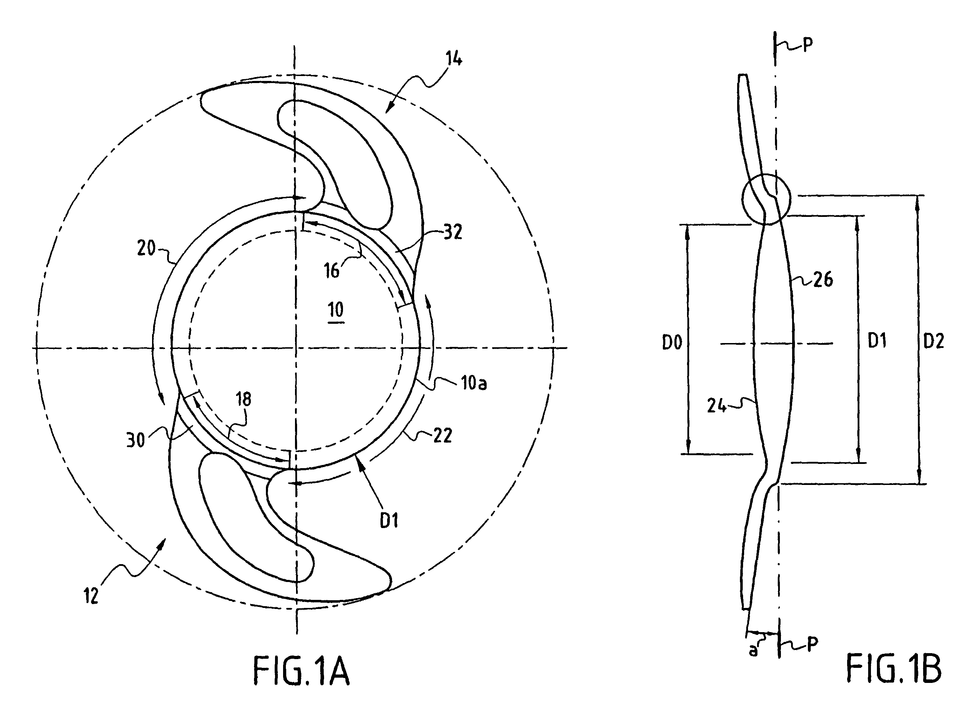

En se référant tout d'abord à la figure 1a sur laquelle on a représenté un implant intraoculaire destiné à être placé dans le sac capsulaire, on voit que celui-ci comporte une partie optique 10 présentant une périphérie circulaire 10a et deux éléments haptiques respectivement référencés 12 et 14. Les éléments haptiques 12 et 14 sont raccordés à la périphérie 10a de la partie optique par des zones de raccordement qui sont repérées par les doubles flèches 16 et 18. On voit également que la périphérie 10a est libre sur le reste de sa longueur repérée par les doubles flèches 20 et 22. Dans ces zones, la paroi latérale 10a de la partie optique 10 est sensiblement cylindrique et raccordée au dioptre postérieur pour former le "bord carré", cette paroi latérale s'étendant vers le dioptre intérieur.

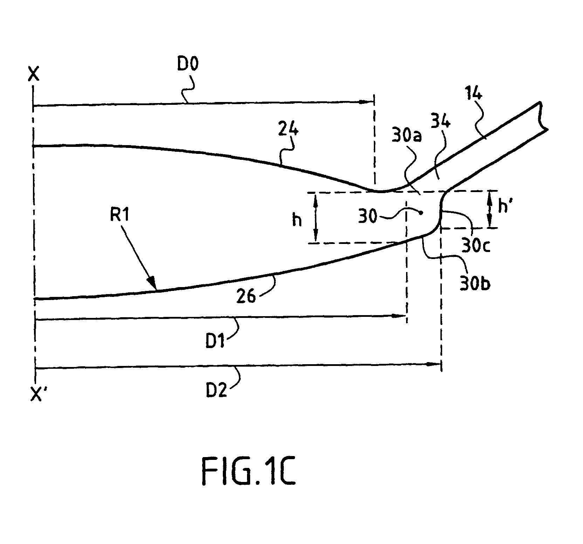

Ainsi qu'on l'a déjà expliqué, il est aisé de prévoir un bord carré pour les zones libres de la périphérie 20 et 22. On va décrire plus en détail en se référant plus particulièrement aux figures 1b et 1c le mode de réalisation selon l'invention qui permet d'obtenir également un bord carré dans les zones de raccordement 16 et 18 tout en évitant une augmentation de l'épaisseur globale de l'implant.

Sur la figure 1b, on a représenté le dioptre antérieur 24 et le dioptre postérieur 26 qui limite la partie optique 10. Le dioptre antérieur 24 est constitué par une calotte sphérique concave ou convexe et limité par un cercle de diamètre D0 centré sur l'axe optique XX' de l'implant. Le dioptre postérieur 26 est limité par un cercle de diamètre D1 qui est de préférence supérieur à D0. Ce cercle de diamètre D1 constitue la limite physique de la partie optique ou bord optique en dehors des zones de raccordement. Le dioptre postérieur 26 est convexe ou plan.

Pour permettre la réalisation du bord carré dans les zones de raccordement 16 et 18, on prévoit dans ces zones de raccordement, comme le montre mieux la figure 1c, des extensions radiales 30 et 32 en regard des zones de raccordement. Chaque extension 30 ou 32 comporte une face antérieure 30a, une face postérieure 30b et une face latérale 30c qui constituent une marche comme on l'expliquera ultérieurement et qui avec la face postérieure 30b constituent le bord carré dans la zone de raccordement. En outre, la face postérieure 30b de l'extension 30 est disposée sur la même calotte sphérique que le dioptre postérieur 26, cette calotte sphérique ayant un rayon R1. La face latérale 30c de l'extension 30 et qui forme la marche et le bord carré est sensiblement disposée sur une surface réglée d'axe X,X' et de diamètre D2 supérieur au diamètre D1 limitant le dioptre postérieur 26. Cette surface réglée peut être assimilée à un cylindre, un tronc, un cône, etc.. Sur les portions de sa périphérie 10a correspondant aux zones libres 20 et 22, le bord optique présente selon la direction de l'axe XX' une longueur h. Dans les zones d'extension 30 et 32, la marche constituée par la paroi latérale 30c présente une longueur h' selon la direction de l'axe XX' qui est légèrement inférieure bien sûr à h.

Sur la figure 1c, on a également représenté le raccordement de l'élément haptique 14 à la périphérie de la partie optique. Le raccordement de l'élément haptique 12 est identique. La racine 34 de l'élément haptique 14 est raccordée sur la face antérieure 30a de l'extension 30 à l'extérieur du dioptre antérieur 24, c'est-à-dire à l'extérieur du cercle de diamètre D0. Ainsi, les propriétés optiques de la partie optique ne sont pas altérées puisque les racines 34 des parties haptiques sont à l'extérieur du dioptre antérieur. En revanche, cette racine étant raccordée sur les faces avant des extensions 30 et 32, elle n'augmente pas l'épaisseur globale de l'implant tout en permettant la présence des marches 30 et 32 qui définissent par leur face latérale 30c et leur face postérieure 30b le bord carré dans ces zones de raccordement.

Selon un mode préféré de réalisation, le diamètre D0 est de l'ordre de 5 mm, le diamètre D1 est de l'ordre de 6 mm et le diamètre D2 est de l'ordre de 6,5 mm. La longueur h' correspondant aux marches dans les zones de raccordement est au moins égale à 120 µm et de préférence comprise entre 120 et 200 µm. Il en résulte que le bord optique de longueur h est légèrement supérieur à cette valeur.

Les expérimentations faites montrent que cette longueur h' de la marche est suffisante pour obtenir le résultat recherché, c'est-à-dire pour bloquer la prolifération des cellules sur la capsule postérieure. Cette longueur, h, h' est liée à la dimension des cellules susceptibles de proliférer sur la capsule postérieure.

Ce résultat est encore amélioré du fait que le rayon R1 est de préférence compris entre 11 et 13 mm, ce qui assure le meilleur contact avec la capsule postérieure assurant ainsi la tension de celle-ci et évitant ainsi les risques de formation de plis. Il va de soi que le rayon du dioptre postérieur étant ainsi défini, la puissance de l'implant sera obtenue en choisissant convenablement le rayon du dioptre antérieur. Cela est possible pour des puissances optiques standard de l'implant.

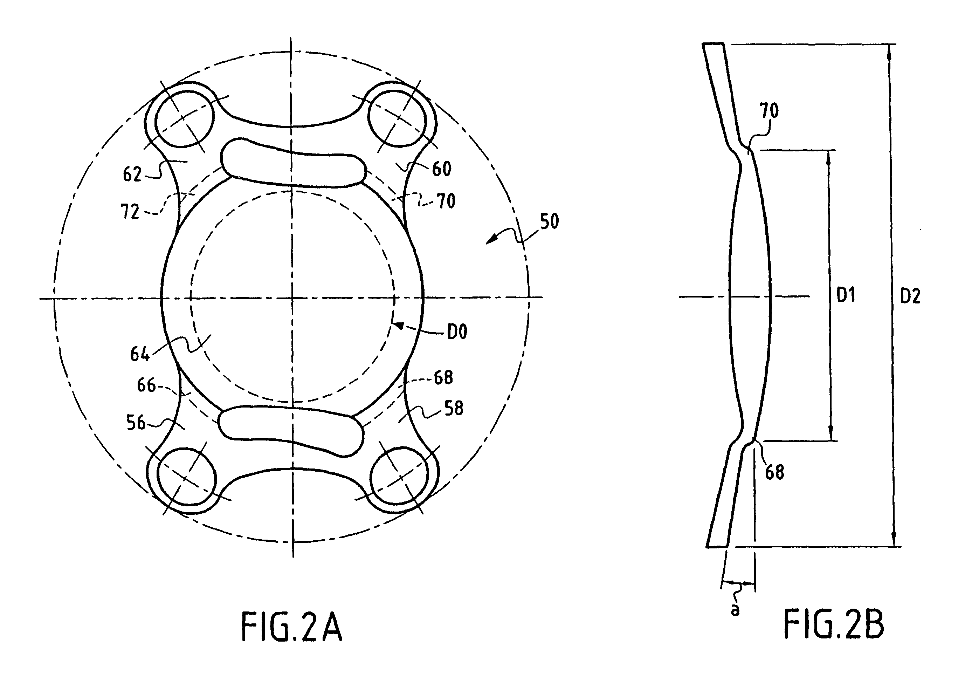

Comme le montre mieux la figure 1b, les bras haptiques 12 et 14 présentent, de préférence avec le plan optique PP', une angulation a comprise entre 5 et 12 degrés. L'angle a est de préférence proche de 10 degrés. Dans le cas de la figure 2b, il vaut 9,5 degrés. Cette angulation des bras haptiques vers l'avant tend à mieux plaquer le dioptre postérieur avec ses extensions contre la capsule postérieure.

Dans ce mode de réalisation, l'implant est monobloc et réalisé en un matériau souple. Chaque élément haptique est constitué par deux bras raccordés entre eux à leur extrémité de contact. Les deux bras comportent une zone de raccordement commune.

L'implant 50 représenté sur les figures 2a et 2b ne se distingue de celui des figures 1a à 1c que par la forme de sa partie haptique. La partie haptique est constituée par deux ensembles haptiques 52 et 54 formés eux-mêmes par deux organes haptiques 56 et 58 et 60, 62, raccordés à la périphérie de la partie optique 64. On a donc dans ce cas quatre zones de raccordement correspondant aux quatre organes haptiques. Dans chaque zone de raccordement, on trouve une extension radiale, les extensions radiales étant référencées de 66 à 72. Les extensions radiales 66 à 72 ont exactement la même forme que les deux extensions radiales 30 et 32 des figures 1a à 1C.

L'implant peut être réalisé soit en un matériau rigide tel que le PMMA, soit en un matériau souple tel que le silicone ou les acryliques. Dans ce dernier cas, on peut utiliser des pHEMA hydrophobes ou hydrophiles.

## Revendications

1. Implant intraoculaire pour sac capsulaire comprenant :
- une partie optique (10) présentant un dioptre antérieur (24) et un dioptre postérieur (26) :
- au moins un élément haptique (12, 14), chaque élément haptique présentant une zone de raccordement (16, 18) à la périphérie de la partie optique qui s'étend sur une portion significative de la périphérie de la partie optique,
- la partie optique comporte en outre, en dehors des zones de raccordement (16, 18), une face latérale cylindrique de diamètre D1 raccordée au dioptre postérieur de la partie optique et parallèle à l'axe optique de l'implant, la longueur de la face latérale selon l'axe étant égale à h,
- le dioptre postérieur est limité par un cercle de diamètre D1,
- et il comprend en outre, dans chaque zone de raccordement, une extension radiale (30, 32) présentant une face antérieure (30a), une face postérieure (30b) et une face latérale (30c) disposée sensiblement sur une surface réglée de diamètre D2 (D2 > D1) et présentant une longueur h' selon la direction de l'axe sensiblement égale à h,
- la face postérieure (30b) de chaque extension est disposée sur la calotte sphérique qui contient le dioptre postérieur,
- chaque élément haptique (12, 14) étant raccordé à la partie optique (10) par la face antérieure (30a) de l'extension correspondante, à l'extérieur du dioptre antérieur (24), chaque extension constitue une marche réalisée par le décalage entre le dioptre postérieur (26) de la partie optique et la zone de raccordement de l'élément haptique, la face latérale (30c) de chaque extension formant une portion de bord carré avec le dioptre postérieur.

2. Implant selon la revendication 1, **caractérisé en ce que** la longueur h et h' des faces latérales (30c) selon la direction de l'axe optique est au moins égale à 150 microns.

3. Implant selon l'une quelconque des revendications 1 et 2, **caractérisé en ce que** la calotte sphérique sur laquelle sont disposés le dioptre postérieur (26) de la partie optique et les faces postérieures des extensions (30b) a un rayon compris entre 11 et 13 mm.

4. Implant selon l'une quelconque des revendications 1 à 3, **caractérisé en ce que** la partie haptique (12, 14) fait avec le plan optique un angle a compris entre 5 et 12 degrés dirigé vers la face antérieure.

5. Implant selon l'une quelconque des revendications 1 à 4, **caractérisé en ce que** le dioptre antérieur (24) est limité par un cercle dont le diamètre D0 est inférieur au diamètre D1.

## Patentansprüche

1. Intraokulares Implantat für den Kapselsack, umfassend:
- einen optischen Teil (10), der einen vorderen Diopter (24) und einen rückwärtigen Diopter (26) aufweist;
- wenigstens ein haptisches Element (12, 14), wobei jedes haptische Element einen Verbindungsbereich (16, 18) an dem Umfang des optischen Teils aufweist, der sich über einen bedeutenden Abschnitt des Umfangs des optischen Teils erstreckt,
wobei das Implantat folgendes aufweist:
- der optische Teil umfaßt des weiteren, außerhalb der Verbindungsbereiche (16, 18), eine seitliche zylindrische Fläche eines Durchmessers D1, die mit dem rückwärtigen Diopter des optischen Teils und parallel zur optischen Achse des Implantats verbunden ist, wobei die Länge der seitlichen Fläche entlang der Achse gleich h ist,
- der rückwärtige Diopter ist durch einen Kreis mit einem Durchmesser D1 begrenzt
- und umfaßt des weiteren in jedem Verbindungsbereich eine radiale Ausdehnung (30, 32), die eine vordere Fläche (30a), eine rückwärtige Fläche (30b) und seitliche Fläche (30c) aufweist, die im wesentlichen auf einer einregulierten Oberfläche mit einem Durchmesser D2 (D2 > D1) angeordnet ist und eine Länge h' aufweist, die entlang der Richtung der Achse im wesentlichen gleich h' ist,
- die rückwärtige Fläche (30b) jeder Ausdehnung ist auf der kugelförmigen Kappe angeordnet, die den rückwärtigen Diopter enthält,
- jedes haptische Element (12, 14) ist mit dem optischen Teil (10) über die vordere Fläche (30a) der entsprechenden Ausdehnung mit der Außenseite des vorderen Diopters (24) verbunden, wobei jede Ausdehnung eine Stufe ausbildet, die durch die Verschiebung zwischen dem rückwärtigen Diopter (26) des optischen Teils und dem Verbindungsbereich des haptischen Elements ausgebildet wird, wobei die seitliche Fläche (30c) jeder Ausdehnung einen viereckigen Randabschnitt mit dem rückwärtigen Diopter ausbildet.

2. Implantat nach Anspruch 1, **dadurch gekennzeichnet, daß** die Länge h und h' der seitlichen Flächen (30c) entlang der Richtung der optischen Achse wenigstens gleich 150 Mikron ist.

3. Implantat nach einem der Ansprüche 1 und 2, **dadurch gekennzeichnet, daß** die kugelförmige Kappe, auf welcher der rückwärtige Diopter (26) des optischen Teils und die rückwärtigen Flächen der Ausdehnungen (30b) angeordnet sind, einen Radius aufweisen, der zwischen 11 und 13 mm liegt.

4. Implantat nach einem der Ansprüche 1 bis 3, **dadurch gekennzeichnet, daß** der haptische Teil (12, 14) mit der optischen Ebene einen Winkel a ausbildet, der zwischen 5 und 12 Grad beträgt und zur vorderen Fläche hin ausgerichtet ist.

5. Implantat nach einem der Ansprüche 1 bis 4, **dadurch gekennzeichnet, daß** der vordere Diopter (24) durch einen Kreis begrenzt wird, dessen Durchmesser D0 kleiner als der Durchmesser D1 ist.

## Claims

1. An intraocular implant for a capsular bag, the implant comprising:
an optical portion (10) presenting an anterior optical surface (24) and a posterior optical surface (26);
at least one haptic element (12, 14), each haptic element presenting a connection zone (16, 18) at the periphery of the optical portion, which zone extends over a significant portion of the periphery of the optical portion, said implant comprising:
outside the connection zones (16, 18), the optical portion further comprises a cylindrical side face of diameter D1 connected to the posterior optical surface of the optical portion and parallel to the optical axis of the implant, the length of the side face along the axis being equal to h;
the posterior optical surface is bounded by a circle of diameter D1;
and in that it further comprises, in each connection zone, a radial extension (30, 32) presenting an anterior face (30a), a posterior face (30b), and a side face (30c) substantially disposed on a ruled surface of diameter D2 where D2 > D1, and presenting a length h' in the direction of the axis, said length h' being substantially equal to h;
the posterior face (30b) of each extension is disposed on the spherical cap containing the posterior optical surface;
each haptic element (12, 14) being connected to the optical portion (10) via the anterior face (30a) of the corresponding extension, on the outside of the anterior optical surface (24), said implant being **characterised in that** each extension constitutes a step formed by the offset between the posterior optical surface (26) of the optical portion and the connection zone of the haptic element, the side face (30c) of each extension forming a square-edged portion with the posterior optical surface.

2. An implant according to claim 1, **characterised in that** the lengths h and h' of the side faces (30c) in the direction of the optical axis are not less than 150 µm.

3. An implant according to claim 1 or 2, **characterised in that** the spherical cap, on which are disposed the posterior optical surface (26) of the optical portion and the posterior faces of the extensions (30b), has a radius lying in the range 11 mm to 13 mm.

4. An implant according to any one of claims 1 to 3, **characterised in that** the haptic portion (12, 14) forms an angle a lying in the range 5° to 12° relative to the optical plane and directed towards the anterior face.

5. An implant according to any one of claims 1 to 4, **characterised in that** the anterior optical surface (24) is bounded by a circle having a diameter D0 that is less than the diameter D1.
